# EUROPEAN PATENT APPLICATION

(11) **EP 4 180 046 A1**
(43) Date of publication of application: **17.05.2023**
(21) Application number: 20944338.1
(22) Date of filing: 31.08.2020
(51) Int. Cl.: A61K 31/6615, A61K 47/32, A61K 47/34, A61P 25/16

(54) **LONG-LASTING RESORBABLE SUBCUTANEOUS IMPLANT WITH SUSTAINED RELEASE OF PRE-CONCENTRATED PHARMACOLOGICALLY ACTIVE SUBSTANCE IN POLYMER FOR THE TREATMENT OF PARKINSON'S DISEASE**

(30) Priority: 07.07.2020 BR 102020013862
(71) Applicant: Luiz Peracchi, Edson, 81230-161 Curitiba (BR)
(72) Inventor: Luiz Peracchi, Edson, 81230-161 Curitiba (BR)
(74) Representative: De Pablos Riba, Juan Ramon
(86) International application number: PCT/BR2020/050347
(87) International publication number: WO 2022/006645

(57) **Abstract**

The present invention privilege application is directed to the medical sector and comprises a long-lasting, resorbable subcutaneous implant with extended release of pre-concentrated pharmacologically active substance in polymer for treatment of Parkinson's disease. The implant is inserted subcutaneously and has continuous release of the active ingredient for an extended period of time. This release aims to ensure an efficient, constant and prolonged serum level of the drug for the adjuvant treatment of Parkinson's disease. The implant of the present invention may have in its constitution only nicotinamide adenine dinucleotide (NADH), but is preferably formed of NADH particles homogeneously dispersed in a bioerodible and bioabsorbable polymer matrix. Implants can have any size, shape or structure that facilitates their manufacture and subcutaneous insertion, however, to obtain a more constant and uniform release of the active ingredient, it is necessary to use geometric shapes that maintain their surface area over time. The implant may have a polymeric coating membrane and the polymer used for the coating must be bioabsorbable and allow the passage of the active substance

## Description

The present application for privilege of invention is directed to the health sector and comprises a long-lasting, resorbable subcutaneous implant with extended release of pharmacologically active substance pre-concentrated in polymer for the treatment of Parkinson's disease.

### State of the art (Background of the invention)

Parkinson's disease (PD) is a chronic and progressive neurodegenerative disease characterized by the presence of multiple monoaminergic dysfunction, including the deficit of dopaminergic, cholinergic, serotonergic and noradrenergic systems.

Parkinson's disease is characterized by the reduction of the functioning of the substantia nigra in the human brain through the death of the neurons present in it. The substantia nigra, located in the midbrain, has two parts, compact and reticular, which have different connections and neurotransmitters. PD is characterized by the degeneration of the compact part and results in decreased availability of dopamine, the main human neurotransmitter present in it.

In normal individuals during the aging process there is a neural loss of 4.7% of the substantia nigra, compared to about 45% in patients with PD. This involvement of the midbrain, in particular of the substantia nigra (compact part), causes a deficiency in dopamine in the nigrostriatal system, which causes a lack of control of the functioning of the basal ganglia, responsible for the pathophysiology of the disease. The motor signs of PD are bradykinesia (decreased voluntary movements), muscle stiffness, tremor and postural instability. Furthermore, adverse consequences such as sleep disorder (sleep fragmentation, sleep apnea, daytime sleepiness and restless legs syndrome), cognitive dysfunction and depression are also observed in practice. All these symptoms have repercussions on a low quality of life and make PD incapacitating and reduce the life expectancy of patients affected by it.

Parkinson's disease affects 5 to 35 new people per 100,000 inhabitants and it is estimated that between 2005 and 2030 the number of people with the disease will double. Added to this increase is the aging of the population and Parkinson's disease becomes a disease with great economic and social impact, having to be correctly treated to improve the quality of life of patients affected by it.

In the classical treatment of Parkinson's disease there are a few dopaminergic drug options, the most widely used being those that act directly on restoring dopamine levels in the brain; on stimulating dopamine receptors or on inhibiting the enzyme monoamine oxidase (MAOI).

The enzyme monoamine oxidase-B (MAO-B) is responsible for the chemical breakdown of dopamine in the brain. Treatment with inhibitors of this enzyme aims to delay the breakdown of dopamine, since the lack of dopamine in the brain causes the adverse symptoms of PD. This is a treatment option indicated for milder cases of the disease, in which the motor symptoms are not yet very intense. The drugs used are selegiline and rasagiline, with doses of 2.5 mg once daily up to a maximum of 5 mg twice daily and 1 mg once daily, respectively.

Other forms of treatment for Parkinson's disease, both for early and more advanced stage, are from its precursor levodopa, also called L-dopa or dopamine agonists. Levodopa is the most potent drug for the treatment of PD and is generally well tolerated by patients. It is usually administered with carbidopa or benserazide, substances that inhibit its extra cerebral decarboxylation, promoting greater availability of levodopa in the brain to be converted to dopamine. The dosage is 100/25 mg levodopa/carbidopa or levodopa/benserazide, three times a day orally, and can reach up to 1,500/375 mg levodopa/carbidopa or levodopa/benserazide per day.

Dopamine agonists, on the other hand, act through bonds with dopaminergic receptors, thereby activating them. The most common dopamine agonists used are: Pramipexole, dosage of 0.125 mg three times a day orally, reaching a maximum of 4.5 mg per day; Pramipexole, with prolonged release, dosage of 0.26, 0.52, 1.05, 2.1 or 3.15 mg per day orally; Ropinirole, dosage of 0.25 mg three times a day orally, reaching a maximum of 24 mg per day; Ropinirole, prolonged release and dosage of 6-24 mg per day orally; and Rotigotine, dosage of 2 mg per day orally, reaching a maximum of 16 mg per day.

However, these conventional treatments have certain unwanted side effects, which are a major disadvantage to the quality of life of patients undergoing treatment for PD. Monoamine oxidase-B enzyme inhibitors may cause a stimulating effect, dizziness, headache and confusion. Treatment with levodopa, on the other hand, can cause nausea, orthostatic hypotension (drop in pressure when standing up), dyskinesia (repetitive involuntary movements) and hallucinations. Treatment with dopamine agonists may cause nausea, orthostatic hypotension, hallucinations, edema, somnolence, and impulse control disorder.

In the article entitled *"Pramipexole* - *a new dopamine agonist for the treatment of Parkinson's disease"-* the authors analyzed data from a survey of 800 patients who were treated with selegiline and vitamin E. In the data analysis, it was possible to observe that 370 of the 800 study participants presented disease progression and began treatment with levodopa, the drug most used in practice. Almost half of this group developed "end of dose deterioration", also known as *wearing off,* a phenomenon in which the drug loses its effectiveness near the end of the interval between doses. About a third of the patients treated with levodopa presented dyskinesia and about a quarter of them presented early signs of locking or freezing, which is the sudden loss of ability to move. These problems occurred during the treatment of about 18 months and demonstrate the limitations of treatment with levodopa orally with regard to side effects.

Despite all these aggravating factors, there is a drug that has a proposed mechanism of action different from those mentioned above for the treatment of PD called Nicotinamide Adenine Dinucleotide (NADH), which is present in the body's cells. In practice, it is observed that treatment with NADH increases the levels of dopamine in the body, in addition to improving the clinical signs of PD. Thus, it is believed that the mechanism of action of NADH in the body is by stimulating the endogenous production of L-DOPA, which is the precursor of dopamine. To transform into dopamine, L-DOPA is metabolically converted from the amino acid tyrosine and this reaction is catalyzed by the enzyme tyrosine hydroxylase (TH). In PD patients the activity of this enzyme is decreased.

In order for the TH enzyme to act in the conversion of dopamine, it is necessary for its coenzyme tetrahydrobiopterin to be available and in sufficient quantity. In patients with PD, there is a 50% decrease in this coenzyme, which may be one of the factors that result in lower TH enzyme activity in these patients. Furthermore, in patients who have an exogenous supply of L-DOPA, the effect of inhibition by feedback of this enzyme may occur.

Thus, an auxiliary way to improve dopamine metabolism is by stimulating TH activity, and this can be done by stimulating biosynthesis of the coenzyme tetrahydrobiopterin. The key enzyme for tetrahydrobiopterin biosynthesis is quinonoid-dihydrobiopterinareductase. This enzyme, in turn, requires a coenzyme called nicotinamide adenine dinucleotide, NADH. Thus, through a succession of reactions, treatment with NADH stimulates the endogenous production of L-DOPA and, consequently, increases dopamine levels in PD patients.

A study entitled *"Nicotinamide adenine dinucleotide (NADH)* - *a new therapeutic approach to Parkinson's disease"* was conducted with 885 patients with Parkinson's disease. Half of the patients were treated with an intravenous infusion of 12.5 mg of NADH in sodium chloride, prepared and filtered just before use and applied every other day. The other half was treated with 5 mg of oral NADH taken every other day, and the treatment lasted 14 days. The results obtained showed that about 80% of patients treated with NADH, in addition to their usual medications, showed at least 10% improvement in symptoms, some of them reaching an improvement of 50%. In addition, two weeks after the end of treatment with NADH, patients presented worsening in their clinical conditions, indicating that the improvement found in treatment with NADH comes from itself. An in vitro study also demonstrated that the use of NADH increased the production of dopamine in cells, showing its ability to directly stimulate the activity of the enzyme tyrosine hydroxylase, increasing its activity by 75%.

Another study entitled *"The coenzyme nicotinamide adenine dinucleotide (NADH) improves the disability of Parkinsonian patients"* was performed with 34 patients with Parkinson's disease, treated with intravenous infusion of 25 mg of NADH in sodium chloride, prepared and filtered just before use and applied every day or on alternate days. Depending on the degree of the disease and the improvement in symptoms, patients were monitored daily according to the duration of treatment, which ranged from 10 to 14 days. All patients treated with NADH improved the clinical symptoms of the disease after 4 days of treatment and the action of NADH lasted between 1 and 2 days after being applied, depending on the severity of the symptoms. Of the 34 patients treated, 61.7% (21 patients) showed more than 30% improvement and the remaining 38.3% (13 patients) showed moderate response to medication, reaching up to 30%. Overall, walking and the ability to push improved considerably, as did posture, speech, and gesture. After NADH withdrawal, the clinical condition of the patients worsened, indicating that NADH was responsible for the observed improvements. Furthermore, 11.8% of the patients showed improvement in symptoms with the use of NADH alone, while the other patients decreased the dosage of the other drugs used by up to 30%.

Referring to a treatment-oriented patent record for Parkinson's disease, US5019561 *(Treatment of Parkinson's disease with NADPH)* mentions the use of the NADPH enzyme cofactor or a salt thereof for treating the pathology. The treatment comprises the application of an intramuscular injection with 25 mg NADPH twice a week. Patients who received this treatment reported significant improvement in motor symptoms. It has been observed that the duration of effect of NADPH is extended when treatment is continued.

Although claiming a treatment for Parkinson's disease through the use of NADPH, the US5019561 record uses intramuscular injections of 25 mg of NADPH twice a week. This type of drug therapy compromises patients' adherence to treatment, as it generates the need for constant visits to the doctor's office to apply the medication, causing inconvenience to the patient. Furthermore, the treatment contained in this patent record is presented in liquid pharmaceutical form, which presents concentrated release of the drug in the body, causing concentration peaks and rapid decay of the amount of active ingredient bioavailable to the patient, distinguishing itself from the reabsorbable subcutaneous implant of the present invention.

In view of the conventional treatment of Parkinson's disease with L-DOPA, NADH has many advantages. Treatment with L-DOPA replaces dopamine, which is low in PD patients, with its precursor (levodopa). However, the replacement of biological substances, produced by the body itself, by external sources, causes a reduction in the natural biosynthesis of this substance, that is, the exogenous medication of L-DOPA inhibits endogenous production. Furthermore, the production of TH enzyme, which assists the conversion of L-DOPA to dopamine, in patients affected by Parkinson's disease is lower than in patients without the disease and decreases further with the exogenous supply of L-DOPA, inhibiting the production of the enzyme. NADH acts in another way in the body, by stimulating the endogenous production of L-DOPA from the activation of the TH enzyme.

In addition to stimulating the endogenous production of L-DOPA, NADH has no disadvantage compared to treatment with L-DOPA, which further destroys the cells of the substantia nigra, caused by the radicals formed by the self-oxidation of the drug. Furthermore, patients who do not respond to treatment with L-DOPA, even at high doses, show improvement when receiving treatment with NADH. In addition, it is known that L-DOPA does not work in a portion of patients with Parkinson's disease, especially after long-term treatment, presenting NADH as a viable and effective alternative.

Turning to the traditional routes of administration of NADH, whether oral, intravenous infusion and intramuscular injection, it can be concluded that there are several disadvantages compared to the innovation proposed in this document. One of the disadvantages is lower therapeutic adherence. According to the World Health Organization, therapeutic adherence is determined by the interaction between the system and the health team, socioeconomic factors, factors related to the patient, treatment and the disease. Adherence to treatment is one of the main factors related to the success or failure of a therapeutic drug approach. The therapeutic outcome is often not as positive as expected due to the patient's conduct. For several reasons, they may not continue the treatment and, therefore, the drug does not produce the expected effect.

Chronic diseases, such as Parkinson's disease, oblige the patient to take at least one drug more than once a day for an indefinite period of time in order to keep the disease under control. The increase in the number of medications taken by the patient per day decreases adherence to treatment by about 20% and the medications used in multiple doses also decrease adherence compared to a single dose.

Furthermore, approximately 50% of patients with chronic diseases do not take their medications as prescribed. The constancy in medication intake among these patients is very low and decreases dramatically after the first six months of therapy. Chronic diseases are long-lasting and can significantly impair the quality of life of those who suffer due to the potential of a high physical, emotional and/or economic burden. The management of these chronic conditions requires continuous attention and commitment of patients and the combination of long-term treatment with the absence of symptoms after medication decreases adherence to treatment in traditional drug approaches.

The oral route is the most susceptible to failure, as it is always dependent on the patient's active participation (or compliance). It is common for patients to make correct use of the drug only around 5 days after consultation with the doctor or when they have acute symptoms. However, in order to obtain all the benefits of drug therapy, it is essential that the patient uses the recommended doses at the time intervals indicated by the physician.

Another disadvantage in the oral use of NADH is the ease in making improper use of the prescribed medication, increasing the risks of underdoses or supraphysiological doses, leading patients to a possible recurrence of symptoms, compromising their general state in the treatment.

In the intravenous infusion and intramuscular injection of NADH, there is a need for a team to do the treatment, since the solution for application must be prepared immediately before use, as it is very sensitive to exposure to light, room temperature and humidity. Furthermore, the infusion is prepared from the mixture of NADH in a solution, and the mixture needs to be filtered before being applied, making unassisted use by the patient unfeasible. Thus, the intravenous and intramuscular treatment of NADH is a costly and complicated activity and requires the patient to go to the responsible clinic at least on alternate days, greatly worsening therapeutic adherence and causing the treatment to be compromised.

The most effective way to increase treatment adherence by patients is to simplify medication dosages. For many chronic diseases, the development of drugs with extended release made it possible to simplify dosages.

Thus, it is possible to find a third option known as NADH bioabsorbable implants or pellets for the treatment of Parkinson's disease. Such implants present extended release of the drug for a long period of time, avoiding concentration peaks and rapid decline of the drug in the body in order to treat the disease, make the treatment independent of the patient taking medication, stimulate the endogenous production of levodopa and avoid the inhibition by feedback of the remaining dopamine, thereby improving its clinical symptoms.

The terms "implant" or "pellet" refer to this pharmaceutical form already consolidated in the official collections of standards for medicines and pharmaceutical substances. They are characterized by being solid and sterile preparations of suitable size and shape for parenteral implantation and release of the active substance(s) over an extended period of time.

The terms "extended release", "slow release" or "sustained release" refer to the form of drug release through the implant, which occurs continuously and gradually for an extended period of time and does not result in an immediate and concentrated release of the drug into the body.

Biodegradable polymers or bioerodible polymers refer to a polymer that degrades *in vivo and* erosion occurs over time concurrently with and/or subsequent release of the therapeutic agent. A biodegradable polymer can be a homopolymer, copolymer, or a polymer compressing more than two polymer units. In some cases, a biodegradable polymer can include mixing two or more homopolymers or copolymers.

Biodegradable implants or bioerodible implants can be understood as implants that have some mechanism that gradually reduces their mass for an extended period of time of release. The forces involved in this mass reduction may be cellular interaction or shear forces on the implant surface. Furthermore, erosion and gradual dissolution of its components is possible. The terms also refer to the total degradation and absorption by the body that occurs at the site where the implants were applied, excluding the need to remove the implants at the end of the treatment.

Referring to patent records directed to resorbable implants, US4957119 *(Contraceptive implant)* mentions an implant of polymeric material that can release a contraceptive agent for a relatively long time when adjusted subcutaneously or locally. The implant comprises an ethylene/vinyl acetate copolymer core material that functions as a matrix for a contraceptive substance, an ethylene/vinyl acetate membrane surrounding the core material, and a contact layer at the interface of the core material and membrane that prevents separation of the core material from the membrane.

Although claiming a resorbable implant, registration US4957119 uses different active substances, its production is carried out by means of extrusion and the release period of the active substance is very long (at least 1 year), distinguishing itself from the resorbable subcutaneous implant of the present invention.

The second registration number US9980850 *(Bioerodible contraceptive implant and methods of use thereof)* describes a bioerodible contraceptive implant and methods of use in the form of a controlled release bioerodible bead for subdermal implantation. The bioerodible sediment provides extended release of a contraceptive agent for an extended period. Bioerosion products are water soluble, bioresorbed or both, avoiding the need for surgical removal of the implant.

As with the first prior art record cited, in this record US9980850 likewise uses distinct active substances the release period of the active substance is very long (from 6 months to 4 years) and the preferred method for manufacturing the granules is the hot melt molding process.

Noting the deficiencies and pre-existing problems in conventional treatments of Parkinson's disease, the present invention aims to treat the pathology, ameliorate the clinical symptoms of patients, stimulate endogenous production of L-DOPA and prevent destruction of the remaining dopamine in the patient by means of a resorbable subcutaneous implant, with prolonged release of the active substance, capable of releasing the drug directly into the bloodstream in order to avoid hepatic metabolism. Another advantage of the present invention is the improvement of the therapeutic adherence of patients, since they do not need to remember to use the drugs or do not need to go to a specialized clinic periodically for the application of the drug. Furthermore, at the end of the treatment with the implants, it is not necessary to remove them, only to reinsert new ones to maintain the treatment.

The development of a treatment that is independent of a patient remembering and patient impairment, as with resorbable NADH implants, is crucial in a disease such as Parkinson's disease, which presents daily obstacles to the well-being and quality of life of patients. Because it is a chronic and neurodegenerative disease, there is a decrease in the patient's ability to collaborate over time due to the limitation that the disease imposes. Furthermore, forgetting to use the medication by patients with Parkinson's disease presents invaluable damage to them, since the half-life of these drugs is low, causing disabling effects even in a short period of time.

### Description of the drawings

For a better understanding of the present invention, the following drawings are attached:
Figure 1 - Representation of the chemical structure of the substance nicotinamide adenine dinucleotide (NADH);
Figure 2 - Dimensional design of the bioabsorbable nicotinamide adenine dinucleotide (NADH)
Figure 3 - Dimensional design of the non-bioabsorbable nicotinamide adenine dinucleotide (NADH) implant.

### Detailed description of the invention

The present application for privilege of invention is a biodegradable nicotinamide adenine dinucleotide (NADH) implant in polymer matrix. The implant is inserted subcutaneously and has continuous release of the active ingredient for an extended period of time. This release aims to ensure an efficient, constant and prolonged serum level of the drug for the adjuvant treatment of Parkinson's disease.

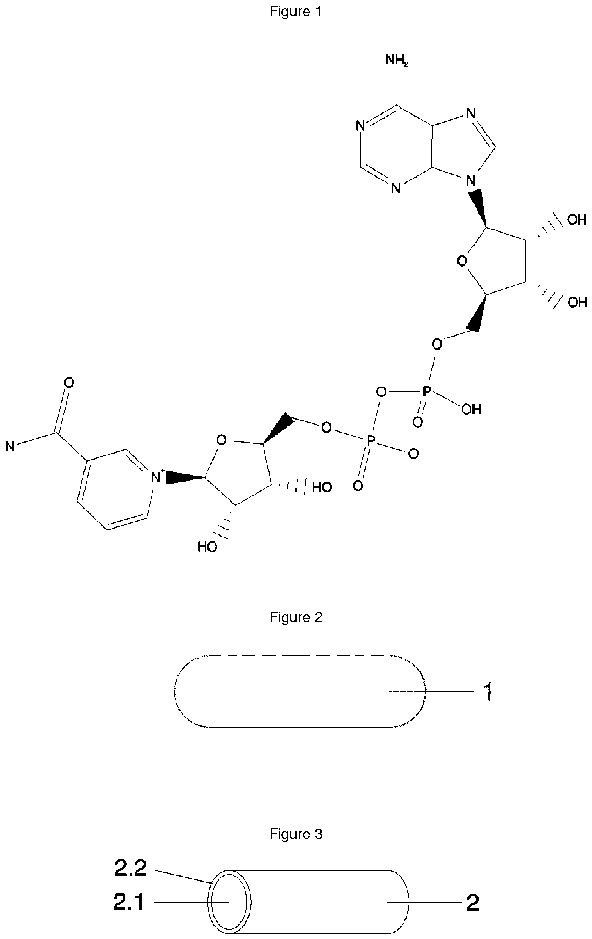

The "active substance", "active ingredient" or "drug" refers to the substance nicotinamide adenine dinucleotide, which has the chemical structure shown in figure 1.

The implant of the present invention may have in its constitution only nicotinamide adenine dinucleotide (NADH), but is preferably formed of NADH particles homogeneously dispersed in a bioerodible and bioabsorbable polymer matrix. Such a polymer matrix may be formed of a polymer or a polymer blend. The amount of nicotinamide adenine dinucleotide (NADH) present in the implant may range from 50 to 250mg per implant and its composition has from 1 to 20% biodegradable polymer in proportion to its weight. Preferably it should have from 80 to 200mg and from 2 to 10% biodegradable polymer in proportion to weight.

The biodegradable polymer used may be: Poly(D-lactic acid), Poly(L-lactic acid), Poly(racemic lactic acid), Poly(glycolic acid), Poly(caprolactone), methylcellulose, ethyl cellulose, hydroxy propyl cellulose (HPC), hydroxy propyl methyl cellulose (HPMC), polyvinylpyrrolidone (PVP), poly(vinyl alcohol) (PVA), polyethylene oxide) (PEO), polyethylene glycol, starch, natural and synthetic gum, and wax.

Implants can have any size, shape or structure that facilitates their manufacture and subcutaneous insertion, however, to obtain a more constant and uniform release of the active ingredient, it is necessary to use geometric shapes that maintain their surface area over time.

Thus, the implant (1) developed and demonstrated in the present application adopts the cylindrical, rod-shaped pattern, provided with straight or rounded tips, with a length between 2 to 25 mm and a diameter of 1 to 6 mm. The schematic drawing of an example implant size (1) is shown in Figure 2.

The manufacture of the nicotinamide adenine dinucleotide (NADH) implant can be made from the addition of 50 to 250 mg of the drug in the solution of the biodegradable polymer matrix chosen in a proportion of 1 to 20% in relation to the weight of the drug, with the formation of a homogeneous mixture. If the polymer solvent is not also a nicotinamide adenine dinucleotide (NADH) solvent, it will be dispersed in the form of particles or suspension, and a mixer can be used to make the solution homogeneous. This solution is then dried and subsequently shaped to the shape of the implant (1) or other desired shape.

Another possible form of making the nicotinamide adenine dinucleotide (NADH) implant is from mixing 50 to 250 mg of the drug and 1 to 20% of the chosen biodegradable polymer matrix with respect to the weight of the drug, in its dry, powdered forms. The drug and the polymer matrix are added in a suitable container and the mixture is homogenized.

The mixture of active ingredients for manufacturing the implant may be shaped from pressure or heat so as not to compromise the efficacy of the drug or degrade the polymeric material. Technique options for implant molding can be: injection molding, hot molding, compression molding, or extrusion molding.

For the present invention, the technique chosen was compression molding. In this technique, the mixture of the active ingredients, in powder form, is added to a mold and there is the application of mechanical force under the mixture, generating the compression of the particles and consequently the molding of the implant in the shape (1). Then there is the filling and sterilization of the nicotinamide adenine dinucleotide (NADH) implant. Its sterilization can be done by heat or gamma rays.

The implant may have a polymeric coating membrane, with a thickness between 0.1 to 0.7 mm. The polymer used for the coating must be bioabsorbable and allow the passage of the active substance. The coating of the implant is preferably done by dipping the implant in a polymer solution. The coating may cover the entire surface of the implant including the edges, only its longitudinal surface with the edges uncoated or coated only on the edges of the implant without coating its length. The polymers that can be used for the coating are: poly(lactic-co-glycolic acid) (PLGA) and copolymers of D, L-lactic acid.

Yet another implant option for treating Parkinson's disease is non-biodegradable implants. Non-biodegradable or non-bioerodible implants (2) (figure 3) have a central core (2.1) formed by a polymeric matrix in the percentage of 1 to 20% in relation to the weight of the drug, in this case 50 to 250 mg of nicotinamide adenine dinucleotide (NADH), the core being surrounded by a non-degradable polymeric membrane (2.2) that controls the release rate of the drug.

The material of manufacture of the polymeric membrane surrounding the implant may be: silicone, urethane, acrylates and their copolymers, polyvinylidene fluoride copolymers, ethylene polyethylene vinyl acetate-vinyl, dimethylpolysiloxane. This membrane has a thickness of 0.2 to 1 mm and is molded in specific equipment. After molding the membrane from the polymeric material there is the insertion of the mixture of nicotinamide adenine dinucleotide (NADH), forming the central core (2.1) of the implant (2). The polymers used in the polymer matrix and the blend adopt the same compounds and process as the bioabsorbable implant.

The release of the drug in this system occurs through diffusion, at a relatively constant rate, and it is possible to change the rate of release of the drug through the thickness or material of that membrane. In this system, it is necessary to remove the implant at the end of the treatment.

The treatment for Parkinson's disease using NADH implants should be defined according to the severity of the symptoms and clinical picture of the patient. The proposed implant lasts approximately 3 to 6 months, and this time is the proposed period between implant insertions. The therapeutic window of NADH in the intramuscular scientific literature is 25 mg twice a week. In total, the amount of active ingredient that can be administered to the patient is 200 mg monthly. Since the treatment with the proposed implant lasts approximately 3 months, the dose used, drawing a parallel with the dose used intramuscularly, is about 600 mg. This dose is equivalent, for example, to 3 implants of 200 mg NADH.

However, to define an individualized treatment for each patient, it is necessary for the physician to evaluate the clinical condition of this patient, use his laboratory tests and clinical analysis of motor signs as support for dosage decision making and start supplementation with partial doses to monitor the remission of symptoms and evolution of the patient's clinical condition. After this first evaluation, the dose can be adjusted by inserting additional implants, if necessary. Furthermore, if rejection or any adverse reaction occurs after insertion of the implant, it may be removed within the first days of treatment.

The use of the implant proposed herein is safe and effective in the treatment of Parkinson's disease, considering that the therapy is independent of the patient's will or discipline for the action of the drug, thus ensuring the maintenance of the dosage and regularity of the treatment. It is noteworthy that patients affected by chronic diseases often discontinue treatment when they notice the reversal of symptoms or when they feel some discomfort due to side effects or adverse reactions, becoming more susceptible to a worsening of their clinical condition.

Thus, the use of these implants in the therapeutic approach prevents discontinuation and guarantees the appropriate treatment, as well as its efficacy. Furthermore, the invention provides patients with Parkinson's disease with a therapy with constant and stable doses of continuous release of the active ingredient, improving their quality of life by improving the adverse clinical symptoms of PD.

Another benefit of this invention is that the release of the drug through the implants occurs directly into the bloodstream, which makes its action much more efficient and avoids a hepatic metabolism of the drug.

Another advantage of the invention is the type and form in which the implant is presented, biodegradable and resorbable, and it is not necessary to remove it after the treatment period, since it leaves no residue in the tissues.

A case study was conducted with a 57-year-old patient with Parkinson's disease, with the onset of the clinical symptoms at 45 years of age, with a history of the disease in the family (brother, paternal uncle and three paternal cousins). The disease history consisted of: unilateral right-sided tremor, bradykinesia, postural instability and muscle stiffness. The patient was medicated with the following medications: Selegiline 5 mg; Pramipexole Dihydrochloride 0.25 mg; Imipramine Hydrochloride 25 mg; Levodopa + Benserazide 100/25 mg; Levothyroxine Sodium 50 mcg; Nortriptyline Hydrochloride 50 mg and Omeprazole 20 mg.

The physical examination indicated cardiac analysis RCR 2T BNF; blood pressure 137 x 86; tremor of the lower and upper limbs, being more accentuated on the right side; slowness of movements to drink a glass of water more accentuated on the right side; slow and dragged gait with anterior inclination, with decreased movements of the arms asymmetrically; movement of the forearms alternately with difficulty in execution and muscle fatigue; irregular writing, with spontaneous movements such as "counting money with your fingers"; muscle stiffness in the forearms and hips; and lack of facial expression.

The patient was treated with 10 resorbable NADH hormone implants (200 mg each). The treatment lasted three months, a period in which it was necessary to reinsert the implants and obtained satisfactory results. The patient evolved with improvement of posture, ambulation and tremors a few hours after the insertion of the implants.

Progressively, with the insertion of the implants, the patient showed improvement in sleep and depression, reporting better conditions to perform domestic tasks and, after a week of insertion of the implants, she no longer saw the need to use antidepressant drugs.

## Claims

1. "Long-acting resorbable subcutaneous implant with extended release of pharmacologically active substance preconcentrated in polymer for the treatment of parkinson's disease", **CHARACTERIZED by** the constitution of the biodegradable implant containing 50 to 250 mg of nicotinamide adenine dinucleotide (NADH) in particles dispersed homogeneously in a bioerodible and bioabsorbable polymer matrix, with the composition of the polymer matrix being 1 to 20% of biodegradable polymer in proportion to the weight of nicotinamide adenine dinucleotide (NADH);

2. "Long-acting resorbable subcutaneous implant with extended release of pharmacologically active substance preconcentrated in polymer for the treatment of parkinson's disease", according to claim 1, **CHARACTERIZED by** the biodegradable polymer being Poly(D-lactic acid), Poly(L-lactic acid), Poly(racemic lactic acid), Poly(glycolic acid), Poly(caprolactone), methylcellulose, ethyl cellulose, hydroxy propyl cellulose (HPC) hydroxy propyl methyl cellulose (HPMC), polyvinylpyrrolidone (PVP), Poly(vinyl alcohol) (PVA), Polyethylene oxide) (PEO), polyethylene glycol, starch, natural and synthetic gum and wax;

3. "Long-acting resorbable subcutaneous implant with extended release of pharmacologically active substance preconcentrated in polymer for the treatment of parkinson's disease", according to claims 1 and 2, **CHARACTERIZED by** the implant adopting the cylindrical pattern (1), rod-shaped with straight or rounded tips and length between 2 to 25 mm and diameter from 1 to 6 mm;

4. "Long-acting resorbable subcutaneous implant with extended release of pharmacologically active substance preconcentrated in polymer for the treatment of parkinson's disease", according to claims 1, 2 and 3, **characterized by** the implant manufacturing process starting with the addition of 50 to 250 mg of nicotinamide adenine dinucleotide (NADH) in the polymer matrix chosen in the proportion of 1 to 20% in relation to the weight of the drug, in its dry forms, in powder, with the addition of the mixture in a container to be homogenized, with the insertion of the powdered active ingredients readily homogenized in a mold to carry out the molding of the implant under mechanical force, compressing the particles and molding the implant in the format (1), with the filling finally occurring;

5. "Long-acting resorbable subcutaneous implant with extended release of pharmacologically active substance preconcentrated in polymer for the treatment of parkinson's disease", according to claims 1, 2 and 3, **CHARACTERIZED by** the implant having a polymeric coating membrane, with a thickness between 0.1 to 0.7 mm, occurring the total coating of the implant including the edges, only its longitudinal surface with the edges uncoated or coated only on the edges of the implant without coating its length, employing as polymeric membrane the polylactic-glycolic acid (PLGA) and copolymers of D,L-lactic acid;

6. "Long-acting resorbable subcutaneous implant with extended release of pharmacologically active substance preconcentrated in polymer for the treatment of parkinson's disease", **CHARACTERIZED by** the implant being presented in non-biodegradable or non-bioerodible form (2), having a central core (2.1) formed by a polymer matrix in the percentage of 1 to 20% in relation to the weight of the drug, in this case 50 to 250 mg of nicotinamide adenine dinucleotide (NADH), the core being surrounded by a non-degradable polymeric membrane (2.2);

7. "Long-acting resorbable subcutaneous implant with extended release of pharmacologically active substance preconcentrated in polymer for the treatment of parkinson's disease", according to claim 6, **CHARACTERIZED by** the material of manufacture of the polymeric membrane surrounding the non-biodegradable implant being silicone, urethane, acrylates and their copolymers, polyvinylidene fluoride copolymers, ethylene polyethylene vinyl acetate-vinyl and dimethylpolysiloxane, said membrane having a thickness of 0.2 to 1 mm and occurring the insertion of nicotinamide adenine dinucleotide (NADH) after its molding, forming the central core (2.1) of the implant (2).
